# EUROPÄISCHE PATENTSCHRIFT

(11) **EP 0 514 687 B1**
(45) Veröffentlichungstag und Bekanntmachung des Hinweises auf die Patenterteilung: **22.02.1995**
(21) Anmeldenummer: 92107099.1
(22) Anmeldetag: 25.04.1992
(51) Int. Cl.: C07C 213/02, C07C 215/76

(54) **Verfahren zur Herstellung von N,N-disubstituierten m-Aminophenolen**
Process for the preparation of N,N-disubstituted m-aminophenols
Procédé pour la préparation des aminophénols N,N-disubstituées

(30) Priorität: 23.05.1991 DE 4116830
(43) Veröffentlichungstag der Anmeldung: 25.11.1992
(73) Patentinhaber: BASF Aktiengesellschaft, 67063 Ludwigshafen (DE)
(72) Erfinder: Hauptreif, Manfred, Dr., W-6700 Ludwigshafen (DE); Reichelt, Helmut, Dr., W-6730 Neustadt (DE)

(56) Entgegenhaltungen:
- EP-A- 0 218 350
- DE-A- 2 900 193
- FR-A- 2 385 683
- PATENT ABSTRACTS OF JAPAN, Band 4, Nr. 89 (C-016), 18. April 1980; & JP-A-55 053 250
- PATENT ABSTRACTS OF JAPAN, Band 11, Nr. 60 (C-405), 1. Oktober 1986; & JP-A-61 221 155
- PATENT ABSTRACTS OF JAPAN, Band 16, Nr. 289 (C-956), 11. März 1992; & JP-A-40 77 460

## Beschreibung

Die vorliegende Erfindung betrifft ein neues Verfahren zur Herstellung von N,N-disubstituierten m-Aminophenolen, ausgehend von Resorcin, das in einem 1. Schritt durch Umsetzung mit primären Aminen in N-monosubstituierte m-Aminophenole übergeführt wird, woraus in einem 2. Schritt durch Behandlung mit Alkylierungsmitteln die N,N-disubstituierten m-Aminophenole hergestellt werden.

Aus der DE-A-2 900 193 ist die Umsetzung von Resorcin mit cyclischen sekundären Aminen in Gegenwart von phosphoriger Säure oder deren Estern bekannt. Man erhält auf diesem Wege N,N-disubstituierte m-Aminophenole, die als substituierte Aminogruppe einen heterocyclischen Rest aufweisen.

Für die Herstellung von solchen N,N-disubstituierten m-Aminophenolen, deren Substituenten an der Aminogruppe beispielsweise Alkyl- oder Cycloalkylgruppen darstellen, werden in der Literatur jedoch andere Syntheserouten empfohlen.

So ist aus der JP-A-61 955/1987 die Herstellung von m-(N,N-Diethylamino)phenol durch Alkylierung von m-Aminophenol mittels Ethylchlorid beschrieben. Aus der JP-A-48 653/1987 oder JP-A-48 654/1987 ist die entsprechende Umsetzung ausgehend von m-(N-Ethylamino)phenol bekannt.

Die jeweiligen Ausgangsprodukte müssen dabei in einer separaten Reaktion, wie beispielsweise in der JP-A-53 250/1980 beschrieben, aus Resorcin und Ammoniak oder primären Aminen hergestellt und zwischengereinigt werden. Solche Zwischenreinigungsschritte sind in der Regel sehr aufwendig (siehe z.B. EP-A-402 935).

Aufgabe der vorliegenden Erfindung war es deshalb, ein neues Verfahren zur Herstellung von N,N-disubstituierten m-Aminophenolen bereitzustellen, das die Zielprodukte auf einfache Weise liefert und bei dem auf eine Reinigung der auftretenden Zwischenprodukte verzichtet werden kann.

Es wurde nun gefunden, daß die Herstellung von m-Aminophenolen der Formel I 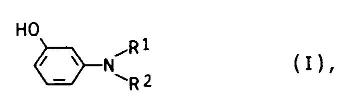
in der
R¹ C₂-C₈-Alkyl oder C₅-C₇-Cycloalkyl und
R² C₁-C₈-Alkyl oder C₅-C₇-Cycloalkyl bedeuten,
vorteilhaft gelingt, wenn man
a) in einem 1. Schritt Resorcin in Gegenwart von phosphoriger Säure, einem Ester der phosphorigen Säure oder deren Mischungen mit einem Amin der Formel II

   R¹-NH₂ (II),

   in der R¹ die obengenannte Bedeutung besitzt, bei einer Temperatur von 180 bis 250°C und einem Druck von 3 bis 40 bar umsetzt und das resultierende Aminophenol der Formel III 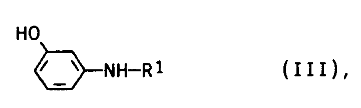 in der R¹ die obengenannte Bedeutung besitzt, ohne Zwischenisolierung
b) in einem 2. Schritt mit einem Alkylierungsmittel der Formel IV

   R²-X (IV),

   in der R² die obengenannte Bedeutung besitzt und X für eine Austrittsgruppe steht, bei einer Temperatur von 60 bis 180°C und bei einem Druck von 1 bis 50 bar in wäßrigem Medium bei einem pH-Wert von 3 bis 7 behandelt.

Alle in der obengenannten Formel I auftretenden Alkylgruppen können sowohl geradkettig als auch verzweigt sein.

Reste R¹ und R² sind z.B. Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, 2-Methylhexyl, Octyl, 2-Methylheptyl, 2-Ethylhexyl, Cyclopentyl, Cyclohexyl oder Cycloheptyl.

Der Rest R² ist weiterhin z.B. Methyl.

Im 1. Schritt ist die Umsetzung von Resorcin mit Aminen der Formel II bevorzugt, in der R¹ C₂-C₆-Alkyl oder Cyclohexyl, vorzugsweise C₄-C₆-Alkyl und insbesondere C₄-Alkyl bedeutet.

Im 2. Schritt ist die Umsetzung der Aminophenole der Formel III mit einem Alkylierungsmittel der Formel IV bevorzugt, in der R² C₁-C₄-Alkyl, insbesondere C₄-Alkyl, bedeutet.

X in Formel IV stellt eine Austrittsgruppe dar. Geeignete Austrittsgruppen sind z.B. Halogen, wie Chlor, Brom oder Iod, Metho- oder Ethosulfat, Benzolsulfonat oder 2- oder 4-Methylbenzolsulfonat.

Für den 1. Schritt des erfindungsgemäßen Verfahrens geeignete Ester der phosphorigen Säure sind z.B. Triphenylphosphit oder C₁-C₈-Trialkylphosphit, wie Trimethylphosphit, Triethylphosphit, Tripropylphosphit, Triisopropylphosphit, Tributylphosphit, Triisobutylphosphit, Tri-secbutylphosphit, Tripentylphosphit, Triisopentylphosphit, Trineopentylphosphit, Trihexylphosphit, Triheptylphosphit, Trioctylphosphit oder Tris(2-ethylhexyl)phosphit.

Die Anwendung von phosphoriger Säure, Triphenylphosphit, C₁-C₄-Trialkylphosphit oder deren Mischungen ist bevorzugt. Besonders bevorzugt ist die Anwendung von phosphoriger Säure.

Der 1. Schritt des erfindungsgemäßen Verfahrens, der sowohl in kontinuierlicher als auch diskontinuierlicher Arbeitsweise vorgenommen werden kann, wird zweckmäßig so durchgeführt, daß man Resorcin, Amin II, gegebenenfalls ein Verdünnungsmittel, phosphorige Säure, einen Ester der phosphorigen Säure oder deren Mischungen in ein Druckgefäß gibt und dann unter Eigendruck oder in Gegenwart von zusätzlichem Inertgas (z.B. Stickstoff) auf eine Temperatur von 180 bis 250°C, vorzugsweise 190 bis 210°C, erhitzt. Der Wert des herrschenden Drucks beträgt in der Regel 3 bis 40 bar, vorzugsweise 10 bis 20 bar.

Nach beendeter Umsetzung, die im allgemeinen 10 bis 20 Stunden in Anspruch nimmt, wird das Reaktionsgemisch abgekühlt und entspannt. Es wird dann, gegebenenfalls nach Abdestillieren des Verdünnungsmittels, direkt, d.h. ohne Zwischenreinigung, für die Alkylierungsreaktion im 2. Schritt verwendet.

Die Menge an phosphoriger Säure, an Ester der phosphorigen Säure oder deren Mischungen liegt in der Regel bei 1 bis 20 Gew.-%, vorzugsweise 2 bis 10 Gew.-% und insbesondere ca. 5 Gew.-%, jeweils bezogen auf das Gewicht der verwendeten Resorcinmenge.

Das für eine optimale Umsetzung benötigte Molverhältnis Amin II : Resorcin liegt im allgemeinen bei 1 : 2 bis 2 : 1, vorzugsweise 1 : 1 bis 1,2 : 1.

Es ist auch möglich, die Umsetzung mit einem größeren Überschuß an Amin II durchzuführen In diesem Fall dient das überschüssige Amin II als Verdünnungsmittel.

Als Verdünnungsmittel können auch inerte organische Lösungsmittel, wie Toluol oder Xylol in Betracht kommen.

Die Durchführung des 1. Schrittes des erfindungsgemäßen Verfahrens im wesentlichen in Abwesenheit eines Verdünnungsmittels ist bevorzugt.

Der 2. Schritt des erfindungsgemäßen Verfahrens, der sowohl in kontinuierlicher als auch diskontinuierlicher Arbeitsweise vorgenommen werden kann, wird zweckmäßig so durchgeführt, daß man das aus dem 1. Schritt resultierende Reaktionsgemisch, enthaltend das Amin der Formel III, vorlegt und zunächst mit Wasser und dem Alkylierungsmittel der Formel IV versetzt.

Dann wird das Gemisch auf eine Temperatur von 60 bis 180°C, vorzugsweise 60 bis 130°C, erwärmt und in dem Maße mit wäßriger Alkalihydroxidlösung versetzt, daß das Reaktionsgemisch stets einen pH-Wert von 3 bis 7, vorzugsweise 4 bis 5, aufweist. Die Alkylierung wird in der Regel bei einem Druck von 1 bis 50 bar vorgenommen. Abhängig vom Siedepunkt des Alkylierungsmittels IV stellt sich dabei ein Druck in diesem Bereich ein.

Nach beendeter Umsetzung, die im allgemeinen 4 bis 9 Stunden in Anspruch nimmt, wird das Reaktionsgemisch abgekühlt und gegebenenfalls entspannt.

Aus dem Reaktionsgemisch kann das Zielprodukt I in üblicher Weise, z.B. durch Abtrennen der organischen Phase, isoliert werden. Die organische Phase kann dann gewaschen und einer Destillation unterworfen werden.

Die Menge an Alkylierungsmittel IV liegt in der Regel bei 1 bis 2 mol, vorzugsweise 1 bis 1,2 mol, bezogen auf 1 mol an Amin II.

Als wäßrige Alkalihydroxidlösung verwendet man beispielsweise wäßrige Natrium- oder Kaliumhydroxidlösung, die eine Konzentration an Natrium- oder Kaliumhydroxid von 5 bis 50 Gew.-%, vorzugsweise 20 bis 30 Gew.-%, aufweist. Bezogen auf 1 Mol Alkylierungsmittel IV, kommen in der Regel 0,9 bis 11 mol Alkalihydroxid zur Anwendung.

Die Menge an Wasser, die bei der Alkylierung zum Amin III gegeben wird, beträgt üblicherweise das zwei- bis fünffache, bezogen auf die Menge an Resorcin.

Es ist auch möglich, die Alkylierungsreaktion unter Schutzgasatmosphäre, z.B. Stickstoffatmosphäre, sowie in Gegenwart von geringen Mengen an Natrium- oder Kaliumiodid und/oder eines oberflächenaktiven Mittels, z.B. einer Benzyltrialkylammoniumverbindung, durchzuführen.

Mittels des neuen Verfahrens gelingt es, N,N-disubstituierte m-Aminophenole in guter Ausbeute und hoher Reinheit herzustellen, wobei das Verfahren technisch einfach abläuft und man auf eine Isolierung und Reinigung des als Zwischenprodukts gebildeten N-monosubstituierten m-Aminophenols verzichten kann. Ein weiterer Vorteil des neuen Verfahrens ist darin zu sehen, daß es auch als Eintopf-Verfahren durchgeführt werden kann.

Bei den N,N-disubstituierten m-Aminophenolen der Formel I handelt es sich um wichtige Zwischenprodukte für die Herstellung von Fluoranen, die als Farbbildner zur Anwendung kommen (siehe z.B. US-A-3 873 573).

Die folgenden Beispiele sollen die Erfindung naher erläutern.

### Beispiel 1

a) 660 g Resorcin, 483 g n-Butylamin und 33 g phosphorige Säure wurden in einem Autoklaven unter Stickstoff für 8 Stunden auf eine Temperatur von 200°C erhitzt, wobei sich ein Druck von 13 bar einstellte. Danach wurde abgekühlt und entspannt.
b) Das in a) erhaltene Reaktionsgemisch (1200 g) wurde unter Stickstoffatmosphäre mit 1,8 l Wasser, 863 g Brombutan, 3,5 g Kaliumiodid und 3 g eines oberflächenaktiven Mittels auf Basis einer Benzyltrialkylammoniumverbindung versetzt und auf 60°C erwärmt. Bei dieser Temperatur wurden 770 g 25 gew.%ige Natronlauge innerhalb von 12 Stunden so zugetropft, daß der pH-Wert des Reaktionsgemischs stets 5 betrug. Anschließend wurde auf 80°C erwärmt und bei dieser Temperatur weitere 125 g 25 gew.%ige Natronlauge innerhalb von 8 Stunden zugetropft, wobei auch hier der pH-Wert des Reaktionsgemischs stets 5 betrug. Danach wurde abgekühlt mit 160 ml Wasser versetzt, die wäßrige Phase abgetrennt und die organische Phase (950 g) mit Wasser gewaschen und nach Abtrennung von Rückstand unter vermindertem Druck (10 mbar) destilliert. Ausbeute an m-Dibutylaminophenol: 850 g (Reinheit, ermittelt durch HPLC: 97 %).

### Beispiel 2

a) 14,52 kg Resorcin, 10,74 kg n-Butylamin und 0,734 kg phosphorige Säure wurden in einem Autoklaven unter Stickstoff für 6 Stunden auf eine Temperatur von 200°C erhitzt, wobei sich ein Druck von 12 bar einstellte. Danach wurde abgekühlt und entspannt.
b) Das in a) erhaltene Reaktionsgemisch (26 kg) wurde unter Stickstoffatmosphäre mit 40 l Wasser, 14,52 kg Chlorbutan und 0,33 kg eines oberflächenaktiven Mittels auf Basis einer Benzyltrialkylammoniumverbindung versetzt und auf 120°C erwärmt. Bei dieser Temperatur wurden 15 l 25 gew.-%ige Natronlauge innerhalb von 20 Stunden so zugetropft, daß der pH-Wert des Gemisches stets 5 betrug. Danach wurde abgekühlt, die wäßrige Phase abgetrennt und die organische Phase mit 25 l Wasser gewaschen und nach Abtrennung von Rückstand unter vermindertem Druck (10 mbar) destilliert.
   Ausbeute an m-Dibutylaminophenol 19,01 kg (Reinheit, ermittelt durch GC: 98,5 %).

### Beispiel 3

a) 11 kg Resorcin, 8,05 kg n-Butylamin und 0,433 kg phosphorige Säure wurden in einem Autoklaven unter Stickstoff für 6 Stunden auf eine Temperatur von 200°C erhitzt, wobei sich ein Druck von 12 bar einstellte. Danach wurde abgekühlt und entspannt.
b) Das in a) erhaltene Reaktionsgemisch (19,5 kg) wurde unter Stickstoffatmosphäre mit 45 l Wasser, 14,52 kg Chlorbutan und 0,33 kg eines oberflächenaktiven Mittels auf Basis einer Benzyltrialkylammoniumverbindung versetzt und auf 120°C erwärmt. Anschließend wurden innerhalb von 6 Stunden 10,4 l 25 gew.-%ige Natronlauge so zugegeben, daß der pH-Wert des Gemisches stets 5 betrug. Die Temperatur wurde in dieser Zeit kontinuierlich von 120°C auf 160°C erhöht. Danach wurde analog Beispiel 2b) aufgearbeitet.
   Ausbeute an m-Dibutylaminophenol 15,5 kg (Reinheit, ermittelt durch GC: 98,6 %).

### Beispiel 4

a) 11 kg Resorcin, 9,2 kg Isopentylamin und 0,55 kg phosphorige Säure wurden in einem Autoklaven unter Stickstoff für 6 Stunden auf eine Temperatur von 200°C erhitzt. Danach wurde abgekühlt und entspannt.
b) Das in a) erhaltene Reaktionsgemisch (20 kg) wurde unter Stickstoffatmosphäre mit 40 l Wasser, 6,5 kg Chlorethan und 0,33 kg eines oberflächenaktiven Mittels auf Basis einer Benzyltrialkylammoniumverbindung versetzt und auf 120°C erwärmt. Bei dieser Temperatur wurden 15 l 25 gew.-%ige Natronlauge innerhalb von 20 Stunden so zugetropft, daß der pH-Wert des Gemisches stets 5 betrug. Danach wurde abgekühlt, die wäßrige Phase abgetrennt und die organische Phase mit 25 l Wasser gewaschen und nach Abtrennung von Rückstand unter vermindertem Druck (10 mbar) destilliert.
   Ausbeute an N-Isopenyl-N-ethyl-m-aminophenol 13,2 kg (Reinheit, ermittelt durch GC: 98 %).

Analog Beispiel 3 werden die in der folgenden Tabelle aufgeführten Verbindungen der Formel 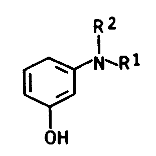
erhalten

| Beispiel-Nr. | R¹ | R² |
|---|---|---|
| 5 | Methyl | Cyclohexyl |
| 6 | Ethyl | Ethyl |
| 7 | n-Propyl | n-Propyl |
| 8 | n-Pentyl | n-Pentyl |

## Patentansprüche

1. Verfahren zur Herstellung von m-Aminophenolen der Formel I 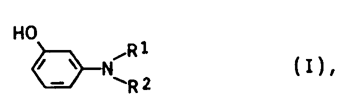 in der
R¹ C₂-C₈-Alkyl oder C₅-C₇-Cycloalkyl und
R² C₁-C₈-Alkyl oder C₅-C₇-Cycloalkyl bedeuten,
dadurch gekennzeichnet, daß man
a) in einem 1. Schritt Resorcin in Gegenwart von phosphoriger Säure, einem Ester der phosphorigen Säure oder deren Mischungen mit einem Amin der Formel II
R¹-NH₂ (II),
in der R¹ die obengenannte Bedeutung besitzt, bei einer Temperatur von 180 bis 250°C und einem Druck von 3 bis 40 bar umsetzt und das resultierende Aminophenol der Formel III 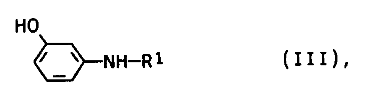 in der R¹ die obengenannte Bedeutung besitzt, ohne Zwischenisolierung
b) in einem 2. Schritt mit einem Alkylierungsmittel der Formel IV
R²-X (IV),
in der R² die obengenannte Bedeutung besitzt und X für eine Austrittsgruppe steht, bei einer Temperatur von 60 bis 180°C und bei einem Druck von 1 bis 50 bar in wäßrigem Medium bei einem pH-Wert von 3 bis 7 behandelt.

2. Verfahren nach Anspruch 1, dadurch gekennzeichnet, daß man Resorcin mit einem Amin der Formel II umsetzt, worin R¹ C₂-C₆-Alkyl oder Cyclohexyl bedeutet.

3. Verfahren nach Anspruch 1, dadurch gekennzeichnet, daß man die Alkylierung mit einem Alkylierungsmittel der Formel IV durchführt, worin R² C₁-C₄-Alkyl bedeutet.

## Claims

1. A process for preparing m-aminophenols of the formula I 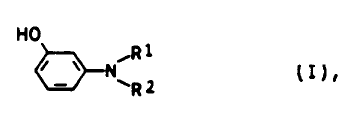 where
R¹ is C₂-C₈-alkyl or C₅-C₇-cycloalkyl and
R² is C₁-C₈-alkyl or C₅-C₇-cycloalkyl,
which comprises
a) in a 1st step reacting resorcinol, in the presence of phosphorous acid, an ester of phosphorous acid or their mixtures, with an amine of the formula II
R¹-NH₂ (II),
in which R¹ has the abovementioned meanings, at from 180 to 250°C and from 3 to 40 bar and treating the resulting aminophenol of the formula III 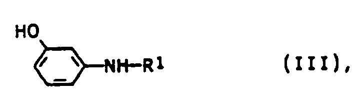 where R¹ has the abovementioned meanings, without intermediate isolation
b) in a 2nd step with an alkylating agent of the formula IV
R²-X (IV),
where R² has the abovementioned meanings and X is a leaving group, at from 60 to 180°C and from 1 to 50 bar in aqueous medium at a pH of 3 to 7.

2. A process as claimed in claim 1, wherein resorcinol is reacted with an amine of the formula II where R¹ is C₂-C₆-alkyl or cyclohexyl.

3. A process as claimed in claim 1, wherein the alkylation is carried out using an alkylating agent of the formula IV where R² is C₁-C₄-alkyl.

## Revendications

1. Procédé de préparation de m-aminophénols de formule I 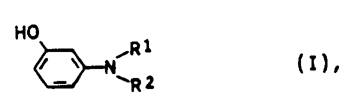 dans laquelle
R¹ représente un reste alkyle en C₂-C₈ ou cycloalkyle en C₅-C₇ et
R² représente un reste alkyle en C₁-C₈ ou cycloalkyle en C₅-C₇,
caractérisé en ce que,
a) dans une 1ère étape, on fait réagir de la résorcine, en présence d'acide phosphoreux, d'un ester de l'acide phosphoreux ou de mélanges de ceux-ci, avec une amine de formule II
R¹-NH₂ (II)
dans laquelle R¹ a la signification susmentionnée, à une température de 180 à 250°C et sous une pression de 3 à 40 bar, et
b) dans une 2ème étape, on traite l'aminophénol de formule III résultant 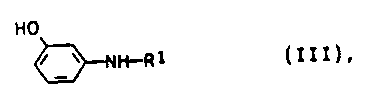 dans laquelle R¹ a la signification susmentionnée, sans isolement intermédiaire, par un agent d'alkylation de formule IV
R²-X (IV)
dans laquelle R² a la signification susmentionnée et X est mis pour un groupement qui s'élimine, à une température de 60 à 180°C et sous une pression de 1 à 50 bar, en milieu aqueux à un pH de 3 à 7.

2. Procédé selon la revendication 1, caractérisé en ce qu'on fait réagir de la résorcine avec une amine de formule II, dans laquelle R¹ représente un reste alkyle en C₂-C₆ ou cyclohexyle.

3. Procédé selon la revendication 1, caractérisé en ce qu'on effectue l'alkylation avec un agent d'alkylation de formule IV dans laquelle R² représente un reste alkyle en C₁-C₄.
